**Europäisches Patentamt**

**European Patent Office**

**Office européen des brevets**

(19)

(11) Publication number: **0 072 194**
**A1**

## (12) EUROPEAN PATENT APPLICATION

(21) Application number: **82304106.6**

(22) Date of filing: **04.08.82**

(51) Int. Cl.³: **A 61 B 1/06**, G 02 B 7/26

(30) Priority: **05.08.81 JP 117199/81 U**

(43) Date of publication of application: **16.02.83**
**Bulletin 83/7**

(84) Designated Contracting States: **AT BE CH DE FR GB IT LI NL SE**

(71) Applicant: **OLYMPUS OPTICAL CO., LTD., 43-2, 2-chome, Hatagaya Shibuya-ku, Tokyo (JP)**

(72) Inventor: **Hashiguchi, Toshihiko, 8-3-15 Seishin, Sagamihara City Kanagawa Prefecture (JP)**

(74) Representative: **Kennedy, Victor Kenneth et al, G.F. REDFERN & CO. Marlborough Lodge 14 Farncombe Road, Worthing West Sussex BN11 2BT (GB)**

(54) **Endoscopes.**

(57) An endoscope wherein a light guide connector (14) is provided to couple in illuminating light fed via a light guide cable from an external light source, the cable being removably connected to the end faces of a bunch of light guide fibres (15) to pass through the endoscope and be projected from the tip of a rigid insertion sheath (11), the connector (14) being formed to be force-fitted to an operating grip portion (12) by engaging a pressed-in part (14a) formed at the base of the light guide connector (14) into a mating opening (12a) in the operating grip portion (12).

ENDOSCOPES

This invention relates to endoscopes of the type wherein a rigid insertion sheath is provided at its rear, operating grip portion with light guide connector to which a light guide cable can be removably fitted.

Recently there has been a growing interest in the use of endoscopes of the type having an elongate rigid insertion sheath to be inserted into a body cavity for observation and diagnosis, or for therapy treatment using a forceps.

In the use of such a hard endoscope, where an affected part such as an internal organ within a body cavity  is to be observed via an observing optical system arranged within the insertion sheath, an illuminating light from an external light source needs to be led via the light guide cable, to be transmitted on through the entrance end surfaces of light guide fibres at the light guide connector to which the light guide cable can be removably fitted, and so transmitted to the tip of the insertion sheath from the operating grip portion via the light guide fibres within the sheath, and will be projected from the tip to illuminate the affected part.

In the known constructions of such endoscopes the light guide connector is fitted to the operating grip portion by screwing, bonding or brazing.

However, fitting by the above mentioned screwing introduces problems, as screwing parts must be provided in both of the operating grip portion and on the light guide connector, so that the working steps will increase, and during the fitting of a connector by screwing, light guide fibres may be twisted and broken.

Fitting by bonding also presents problems, as it is difficult to positively fix the light guide mouthpiece to the operating grip portion with adequate strength and, in bonding, there have been problems that such pretreatment as degreasing must be effectively carried out.

Fitting by brazing or the like presents problems due to the temperature required in the case of brazing, as the bonding agent used to form the light guide fibres into a bunch may be scorched and lose its function as a bonding agent, and in any case the scorched portion will absorb illuminating light, and reduce efficiency so that the light will not be well transmitted.

The present invention relates to a hard endoscope wherein a light guide cable transmitting an illuminating light of an external light source is connected at the end with light guide fibres as illuminating light transmitting means through a light guide mouthpiece, characterised in that an opening is formed on the side of an operating part and a pressed-in part which can be pressed and fixed in the above mentioned opening is formed on the base side of the light guide mouthpiece.

One object of the present invention is to provide an endoscope wherein a light guide connector can be fitted to an operating grip portion by a simple structure, avoiding the above-mentioned problems at a low cost, whilst ensuring that the connector can be fitted to an operating grip portion without a risk of breaking light guide fibres.

According to the present invention, there is provided an endoscope of the type wherein a rigid insertion sheath contains light guide fibres extending from its tip to a connector on an operating grip portion, said connector being coupled, when in use, to a light guide cable leading an external illuminating light source, characterised in that an opening is formed on the side of the operating grip portion the base end of the light guide connector is formed as a pressed-in part so as to be force-fitted into said opening to fix the light guide connector to the operating grip portion.

The invention will now be described with reference to the drawings, in which:-

Figure 1 is an elevation showing a known construction of an endoscope;

Figure 2 is a sectional view showing details of the known light guide connector fitted to an operating grip portion in the conventional endoscope;

Figure 3 is a sectional view showing part of one exemplary embodiment of the present invention;

Figure 4 is a sectional view on line A-A' in Figure 3;

Figure 5 is a sectional view showing part of a second exemplary embodiment of the present invention;

Figure 6 is a sectional view showing part of a third exemplary embodiment of the present invention; and

Figure 7 is a sectional view on line B-B' in Figure 6.

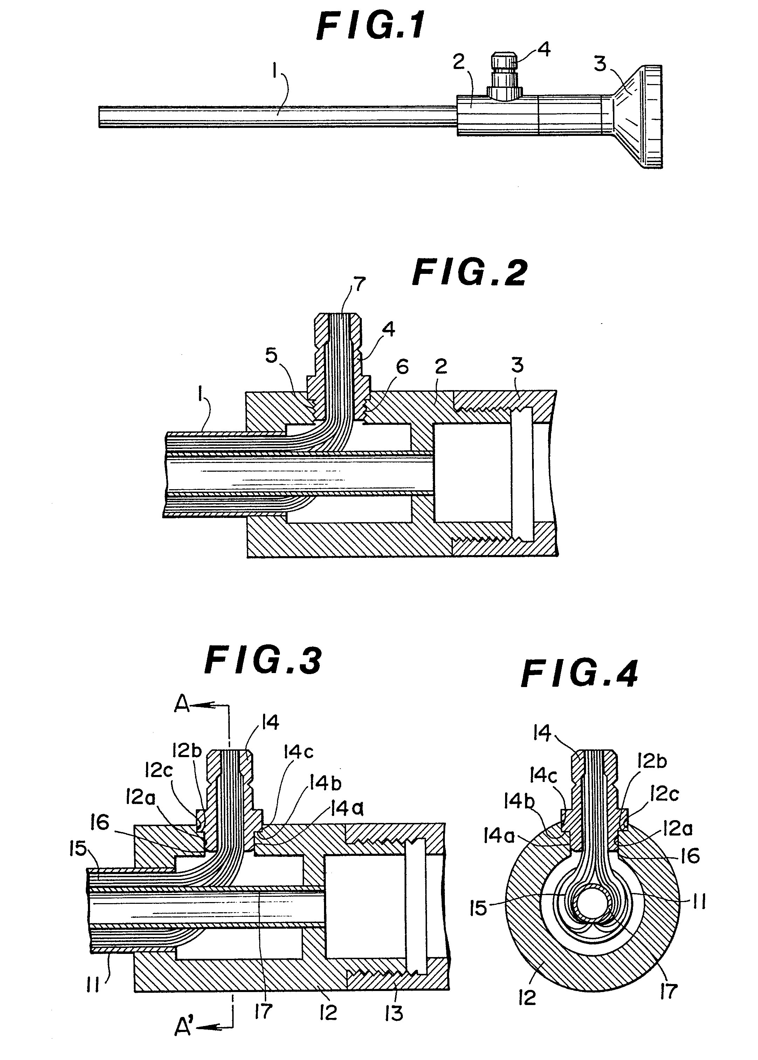

Before describing the exemplary embodiments of the present invention, the known construction of a conventional hard endoscope will be explained with reference to Figures 1 and 2.

As shown in Figure 1, the endoscope comprises an insertion sheath 1 to be inserted into a body cavity, an operating grip portion 2 for operation, and an eyepiece 3 for observation. A light guide connector 4 is provided on the grip portion 2 to connect a light guide cable (not shown) connected to an external light source (not shown).

As shown in Figure 2, a male screw thread 5 is cut in the connecting part of the light guide connector 4, and a female screw thread 6 is cut in the wall of a hole in the operating grip portion 2, and these screw threads 5 and 6 are screwed together to secure the light guide connector 4 to the operating grip portion 2. A light guide fibre bunch 7 is inserted to extend from the above-mentioned light guide connector 4 to the tip of the insertion sheath 1. A light guide cable leading an illuminating light of an external light source (not illustrated) is removably fitted to the connector by a click-stop groove on the above-mentioned light guide connector 4 so that the illuminating light may be transmitted on to be projected at the tip of the insertion sheath.

However, when screwing the components together, as the female screw thread and male screw thread must be cut in the operating grip portion 2 and light guide connector 4 respectively, the working steps are increased, the light guide fibre bunch 7 may be twisted and broken as the light guide connector 4 is screwed in, and in cases where a stainless steel is used for the light guide connector 4 to increase resistance to corrosion, it will be difficult to cut screw threads, the tool (blade) will be worn excessively and the space for the light guide fibres will be decreased by the need to provide for the screw thread in the connector wall.

The present invention is intended to avoid the problems of the above-mentioned conventional example and of the above described prior art.

Figures 3 and 4 show a first exemplary embodiment of the present invention. A rigid insertion sheath 11 of an endoscope is mounted on an operating grip portion 12 that has an eyepiece 13 at its rear end, and a light guide connector 14 containing a bunch of light guide fibres 15. The insertion sheath 11 of the endoscope is fitted to the operating grip portion 12 and the eyepiece 13 is screwed to this same part 12. The light guide connector 14 holding the light guide fibres 15 and forming a light entry port to the endoscope is connected at the outer periphery of the operating grip portion 12. In order to connect the operating grip portion 12 and the light guide connector 14 together, an opening 12a is provided in the operating grip portion 12 and a pressed-in part 14a to be force-fitted into opening 12a is provided at the base of the light guide connector 14.

Further, in cases where the outer cross-section of the operating grip portion 12 is circular, a flat butting surface 12b is formed as a contact and engagement surface below an enlarged diameter entry portion 12c in the opening 12a of the operating grip portion 12. Both the pressed-in part 14a and a butting part 14b of a flange 14c at the rear of the pressed-in part 14a firmly and precisely engage, locate and secure the connector 14 when fitted to the operating grip portion 12. The caliber difference (so-called interference) between the internal diameter of the opening 12a and the external diameter of the pressed-in part 14a is about 10 to 20$\mu$m. With this low caliber difference, the pressed-in part 14a can be firmly pressed into the opening 12a, the light guide connector 14 then being securely fitted to the operating grip portion 12, and the butting parts 12b and 14b being brought into close contact with each other ensures that the position of the light guide connector 14 is correct in the vertical direction (the direction normal to the respective point on to the outer surface of the operating grip portion 12) and shields any clearance between the operating grip portion 12 and light guide connector 14 at the covering parts 12c and 14c. A taper 16 may be made by chamfering the peripheral edge of the extreme tip of the pressed-in part 14a of the light guide connector 14 to facilitate fitting the pressed-in part 14a into the opening 12a. An inner tube 17 provided in the sheath 11 contains an optical system, which is not shown in detail.

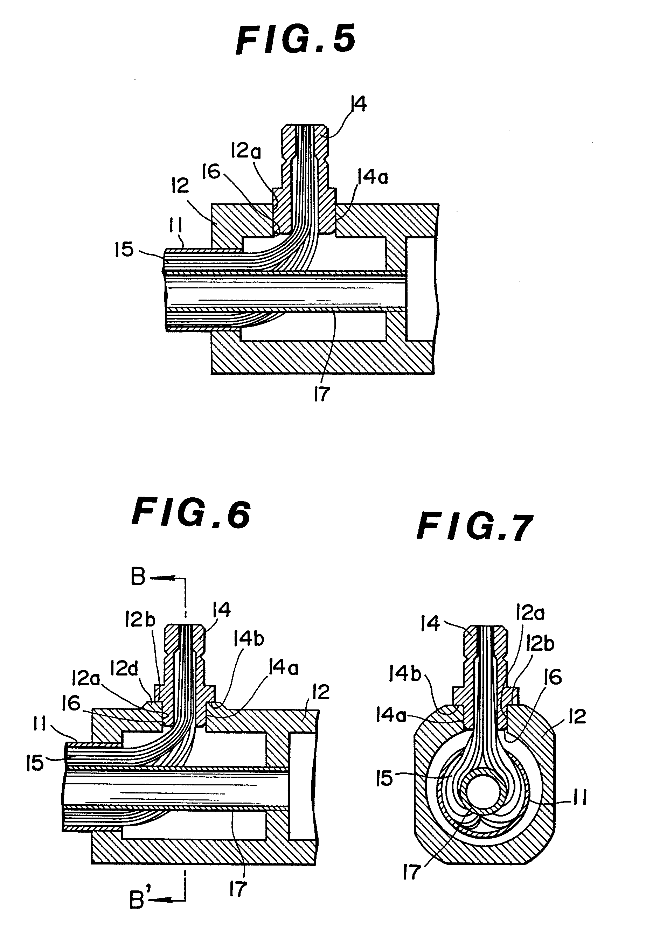

Figure 5 shows the second exemplary embodiment of the present invention, in which the butting parts 12b and 14b for determining the position in the vertical direction in the first embodiment are omitted, and the light guide connector 14 is fitted in the operating grip portion 12 so that the opening 12a is only engaged by the pressed-in part 14a. The position in the direction normal to the outer surface of the operating grip portion 12 is determined by use of a positioning jig, so that the outer wall surfaces 12c and 14c are not required. Even in the case of such a simplified fitting form, the caliber difference between the inside diameter of the opening 12a and the outside diameter of the pressed-in part 14a can be about 10 to 20$\mu$m, as the pressed-in part 14a is securely fitted by this caliber difference because of the greater length of mutual engagement, compared with the first embodiment.

Figures 6 and 7 show the third embodiment of the present invention, in which the outer surface of the operating grip portion 12 is approximately square, and is provided with a square opening 12a. An elevated part 12d defining a flat outer peripheral surface is provided on the operating grip portion in the embodiment shown in Figure 6, and has the opening 12a formed in it. The pressed-in part 14a of the light guide connector 14 is force-fitted into the opening 12a until a butting part 14b at the front end of a flange formed on the light guide connector 14 is brought into close contact with the butting part 12b at the outer peripheral end of the opening 12a, so that the light guide connector 14 is positioned and held in the vertical direction. Even in this fitting form, the pressed-in part 14a is pressed and fitted in the opening 12a on the basis of the same caliber difference as the above-described embodiments.

Thus the pressed-in part 14a of the light guide connector 14 is pressed into close contact with a predetermined part of the opening 12a of the operating grip portion 12 so as to fit and fix the light guide connector 14 to the operating grip portion 12.

In this third embodiment, the light guide mouthpiece 14 is pressed into the opening 12a formed in the operating part 12 until the butting part 14b of the light guide mouthpiece 14 contacts the butting part 12b on the flat end surface at the outer periphery of the opening 12a of the elevated part 12d, so as to be positioned and fixed in the vertical direction. However, as in the first embodiment, butting surfaces 12b and 14b may be formed internally in the side wall of the opening 12a (in such case, the outer covering parts 12c and 14c will also be formed). Further, it is needless to say that, whatever the overall shape of the elevated part 12d, only that part immediately surrounding the opening 12a need be shaped to be flat.

The position of the light guide mouthpiece 14 in the vertical direction (as drawn) can be determined by using a jig, during pressing and fixing, as described with reference to the second embodiment, and butting parts, 12b and 14b, together with associated covering parts 12c and 14c can then be omitted.

The cross-sections of the opening 12a, pressed-in part 14a, and any flange part provided with a butting surface 14b must obviously be matched, but may be circular, or square, or of any other shape required to meet a particular problem.

It is apparent that various modifications can be formed in a wide range without deviating from the spirit and scope of the present invention, which is defined in the claims.

CLAIMS:-

1.    An endoscope of the type wherein a rigid insertion sheath contains light guide fibres extending from its tip to a connector on an operating grip portion, said connector being coupled, when in use, to a light guide cable leading an external illuminating light source, characterised in that  an opening is formed on the side of the operating grip portion the base end of the light guide connector is formed as a pressed-in part so as to be force-fitted into said opening to fix the light guide connector to the operating grip portion.

2.    An endoscope according to Claim 1, characterised in that a flange part is formed at the rear end of the pressed-in part of the light guide connector so as to contact and engage with the grip portion outer periphery around the opening when the pressed-in part is fitted into the opening.

3.    An endoscope according to Claim 1, characterised in that a flange part is formed at the rear end of the pressed-in part of the light guide connector and the outer end of the opening  is of enlarged diameter, so as to contact and engage with said flange part.

4.    An endoscope according to Claim 1, characterised in that the grip portion surface around the periphery of the opening has a flat face  portion, and a flange is formed at the end of the pressed-in part of the light guide connector so as to contact and engage with said flat face portion when the connector part is fitted.

½    0072194

FIG.1

FIG.2

FIG.3

FIG.4

## FIG.5

## FIG.6

## FIG.7

European Patent Office

**EUROPEAN SEARCH REPORT**

0072194

Application number

EP 82 30 4106.6

## DOCUMENTS CONSIDERED TO BE RELEVANT

| Category | Citation of document with indication, where appropriate, of relevant passages | Relevant to claim |
|---|---|---|
| Y | GB - A - 1 463 350 (BOWTHORPE-HELLERMANN LTD.) <br> * page 1, lines 17 to 32 * <br> -- | 1-4 |
| Y | GB - A - 1 365 723 (CAVIS CAVETTI ISOLATI S.P.A.) <br> * page 1, lines 35 to 46 * <br> -- | 1-4 |
| A | US - A - 4 014 098 (VICON PRODUCTS CORP.) <br> * column 2, lines 53 to 67 * <br> ---- | 1 |

**CLASSIFICATION OF THE APPLICATION (Int. Cl. 3)**

A 61 B 1/06

G 02 B 7/26

**TECHNICAL FIELDS SEARCHED (Int.Cl. 3)**

A 61 B 1/00

G 02 B 5/14

G 02 B 5/16

G 02 B 7/26

G 02 B 23/08

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 15-09-1982 | BOTTERILL |

EPO Form 1503.1 06.78